(19) Europäisches Patentamt
European Patent Office
Office européen des brevets

(11) Veröffentlichungsnummer: **0 521 500 A1**

(12) **EUROPÄISCHE PATENTANMELDUNG**

(21) Anmeldenummer: **92111196.9**

(51) Int. Cl.5: **C07D 521/00**, A01N 47/36

(22) Anmeldetag: **02.07.92**

(30) Priorität: **05.07.91 DE 4122306**

(43) Veröffentlichungstag der Anmeldung: **07.01.93 Patentblatt 93/01**

(84) Benannte Vertragsstaaten: **AT BE CH DE DK ES FR GB GR IT LI NL PT SE**

(71) Anmelder: **HOECHST AKTIENGESELLSCHAFT**
**Postfach 80 03 20**
**W-6230 Frankfurt am Main 80(DE)**

(72) Erfinder: **Kehne, Heinz, Dr.**
**Iltisweg 7a**
**W-6238 Hofheim am Taunus(DE)**
Erfinder: **Haaf, Klaus, Dr.**
**Breslauerstrasse 57**
**W-6233 Kelkheim am Taunus(DE)**
Erfinder: **Bauer, Klaus, Dr.**
**Doorner Strasse 53d**
**W-6450 Hanau 7(DE)**
Erfinder: **Bieringer, Hermann, Dr.**
**Eichenweg 26**
**W-6239 Eppstein/Ts.(DE)**

(54) **Salze von Pyridylsulfonylharnstoffen als Herbizide und Pflanzenwachstumsregulatoren, Verfahren zu ihrer Herstellung und ihre Verwendung.**

(57)

Verbindungen der Formel (I)

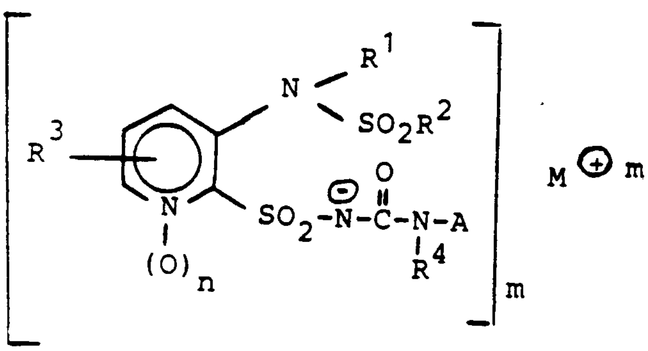

(I)

worin

| | |
|---|---|
| $R^1$ | H oder (jeweils subst.) Alkyl, Alkenyl, Alkinyl, Alkylsulfonyl, Phenylsulfonyl, Alkoxy oder Alkylsulfonyl, |
| $R^2$ | (subst.) Alkyl, (subst.) Phenyl oder Dialkylamino oder |
| $R^1$ und $R^2$ | gemeinsam eine (subst.) $C_3$-$C_4$-Alkylenkette, |
| $R^3$ | H, Alkyl, Haloalkyl, Hal, $NO_2$, CN, Alkoxy, Haloalkoxy, Alkylthio, Alkoxyalkyl, Alkoxycarbonyl, Mono- oder Dialkylamino, Alkylsulfonyl, Alkylsulfinyl, (subst.) Amidosulfonyl oder (subst.) Carbonamid, |
| $R^4$ | H oder $CH_3$, n = O oder 1, m = 1 oder 2 |

EP 0 521 500 A1

A 4,6-disubstituiertes Pyrimidinyl (s. Anspruch 1)
M ein- oder zweiwertiges Kation (s. Anspruch 1) bedeuten,
sind als Herbizide und Pflanzenwachstumsregulatoren geeignet. Die Herstellung erfolgt z.B.aus dem entsprechenden Sulfonylharnstoff durch Umsetzung mit einem Alkoholat, Carbonat oder Amin.

Es ist bekannt, daß einige 2-Pyridylsulfonyharnstoffe herbizide und pflanzenwachstumsregulierende Eigenschaften besitzen; vergleiche EP-A-13 480 (US-A-4 435 206), EP-A-272 855 (US-A-4 838 926), EP-A-84 224 (US-A-4 629 494), US-A-4 421 550, EP-A-103 543 (US-A-4 579 583), US-A-4 946 494, US-A-4 487 626, EP-A-125 864 (US-A-4 723 991), WO 88/04297, EP-A-178 101 (US-A-4 756 742).

In der PCT-Patentanmeldung Nr. PCT/EP 90/02308 (WO-91/10660) wurden bereits 2-Pyridylsulfonylharnstoffe mit speziellen Resten in 3-Position des Pyridylrestes als Herbizide und Pflanzenwachstumsregulatoren vorgeschlagen. Salze der Verbindungen sind dort nur allgemein als alternative Einsatzformen der Herbizide erwähnt.

Es wurde nun gefunden, daß bestimmte landwirtschaftlich einsetzbare Salze von 2-Pyridylsulfonylharnstoffen mit speziellen Resten in 3-Position des Pyridylrestes besonders gut als Herbizide und Wachstumsregulatoren geeignet sind.

Gegenstand der vorliegenden Erfindung sind Verbindungen (Salze) der Formel (1),

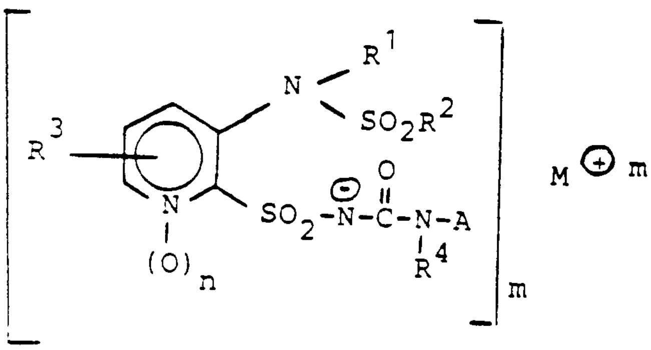

(I)

worin

| | |
|---|---|
| $R^1$ | H, $(C_1\text{-}C_6)$Alkyl, das unsubstituiert oder durch ein oder mehrere Reste aus der Gruppe Halogen, $(C_1\text{-}C_4)$Alkoxy, $(C_1\text{-}C_4)$Alkylthio, $(C_1\text{-}C_4)$Alkylsulfinyl, $(C_1\text{-}C_4)$-Alkylsulfonyl, $[(C_1\text{-}C_4)$-Alkoxy]-carbonyl und CN substituiert ist, $(C_3\text{-}C_6)$Alkenyl, das unsubstituiert oder durch ein oder mehrere Halogenatome substituiert ist, $(C_3\text{-}C_6)$-Alkinyl, das unsubstituiert oder durch ein oder mehrere Halogenatome substituiert ist, $(C_1\text{-}C_4)$Alkylsulfonyl, das unsubstituiert oder durch ein oder mehrere Halogenatome substituiert ist, Phenylsulfonyl, wobei der Phenylrest unsubstituiert oder durch ein oder mehrere Reste aus der Gruppe Halogen, $(C_1\text{-}C_4)$Alkyl und $(C_1\text{-}C_4)$Alkoxy substituiert ist, $(C_1\text{-}C_4)$Alkoxy oder $[(C_1\text{-}C_4)$Alkyl]-carbonyl, das unsubstituiert oder durch ein oder mehrere Halogenatome substituiert ist, |
| $R^2$ | $(C_1\text{-}C_4)$Alkyl, das unsubstituiert oder durch ein oder mehrere Halogenatome substituiert ist, Phenyl, wobei der Phenylrest unsubstituiert oder durch ein oder mehrere Reste aus der Gruppe Halogen, $(C_1\text{-}C_4)$Alkyl und $(C_1\text{-}C_4)$Alkoxy substituiert ist, oder Di-$[(C_1\text{-}C_4)$-alkyl]amino oder |
| $R^1$ und $R^2$ | gemeinsam eine Kette der Formel $-(CH_2)_o-$, wobei die Kette noch durch 1 bis 4 $(C_1\text{-}C_3)$-Alkylreste substituiert sein kann und o 3 oder 4 bedeutet, |
| $R^3$ | H, $(C_1\text{-}C_4)$Alkyl, vorzugsweise $(C_1\text{-}C_3)$Alkyl, $(C_1\text{-}C_3)$Haloalkyl, Halogen, $NO_2$, CN, $(C_1\text{-}C_3)$-Alkoxy, $(C_1\text{-}C_3)$Haloalkoxy, $(C_1\text{-}C_3)$Alkylthio, $(C_1\text{-}C_3)$Alkoxy-$(C_1\text{-}C_3)$alkyl, $[(C_1\text{-}C_3)$Alkoxy]-carbonyl, $(C_1\text{-}C_3)$Alkylamino, Di$[(C_1\text{-}C_3)$alkyl]-amino, $(C_1\text{-}C_3)$-Alkylsulfinyl, $(C_1\text{-}C_3)$-Alkylsulfonyl, $SO_2NR^aR^b$ oder $C(O)NR^aR^b$, |
| $R^a$,$R^b$ | unabhängig voneinander H, $(C_1\text{-}C_3)$Alkyl, $(C_3\text{-}C_4)$-Alkenyl oder Propargyl oder zusammen $-(CH_2)_4-$, $-(CH_2)_5-$ oder $-CH_2CH_2OCH_2CH_2-$ |
| $R^4$ | H oder $CH_3$, |
| n | null oder 1, |
| m | 1 oder 2, |
| A | einen Rest der Formel |

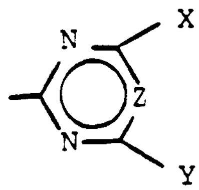

X, Y — unabhängig voneinander H, Halogen, $(C_1\text{-}C_3)$Alkyl, $(C_1\text{-}C_3)$Alkoxy oder $(C_1\text{-}C_3)$Alkylthio, wobei die vorgenannten alkylhaltigen Reste unsubstituiert oder ein- oder mehrfach durch Halogen oder ein- oder zweifach durch $(C_1\text{-}C_3)$Alkoxy oder $(C_1\text{-}C_3)$Alkylthio substituiert sind, ferner einen Rest der Formel $NR^5R^6$, $(C_3\text{-}C_6)$-Cycloalkyl, $(C_2\text{-}C_4)$Alkenyl, $(C_2\text{-}C_4)$-Alkinyl, $(C_3\text{-}C_4)$-Alkenyloxy oder $(C_3\text{-}C_4)$Alkinyloxy,

Z — CH oder N,

$R^5$ und $R^6$ — unabhängig voneinander H, $(C_1\text{-}C_3)$Alkyl oder $(C_3\text{-}C_4)$Alkenyl und

M — ein Atom aus der Gruppe der Alkalimetalle, Erdalkalimetalle oder eine Gruppe der Formel M1,

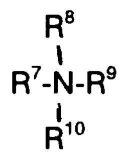 (M1)

worin $R^7$, $R^8$, $R^9$ und $R^{10}$ unabhängig voneinander für H, $(C_1\text{-}C_{12})$Alkyl, $(C_3\text{-}C_6)$Alkenyl, $(C_3\text{-}C_6)$Alkinyl, $(C_3\text{-}C_8)$-Cycloalkyl oder Phenyl, wobei die 5 letztgenannten Reste jeweils unabhängig voneinander unsubstituiert oder durch ein oder mehrere Reste aus der Gruppe Halogen, $(C_1\text{-}C_4)$Alkyl, Hydroxy, $(C_1\text{-}C_4)$Alkoxy, Thiol, $(C_1\text{-}C_4)$Alkylthio, $[(C_1\text{-}C_4)$-Alkoxy]-carbonyl und gegebenenfalls substituiertes Phenyl substituiert sind, stehen oder zwei der Reste $R^7$ bis $R^{10}$ gemeinsam für eine gesättigte oder ungesättigte Kette von 3-7 C-Atomen, wobei 1-2 C-Atome durch Atome aus der Gruppe O, N oder S ersetzt sein können und die Kette durch 1-3 $(C_1\text{-}C_4)$Alkylreste substituiert sein kann, stehen und die übrigen zwei Reste die vorher genannten Bedeutungen für einzelne Reste $R^7$ bis $R^{10}$ haben,

bedeuten.

In der Formel (I) und im folgenden können Alkyl-, Alkoxy-, Haloalkyl-, Alkylamino- und Alkylthioreste sowie die entsprechenden ungesättigten und/oder substituierten Reste jeweils geradkettig oder verzweigt sein. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl. Halogen bedeutet Fluor, Chlor, Brom oder Jod; Haloalkyl bedeutet Alkyl, das durch ein oder mehrere Atome aus der Gruppe Halogen substituiert ist; Haloalkyl ist beispielsweise $CF_3$, $CHF_2$, $CH_2CF_3$. Gegebenenfalls substituiertes Phenyl bedeutet vorzugsweise unsubstituiertes Phenyl oder Phenyl, das durch ein oder mehrere, vorzugsweise 1 bis 3 Reste aus der Gruppe Halogen, Alkyl, Alkoxy, Nitro, Cyano, Alkoxycarbonyl, Alkamoyl, Carbamoyl, Mono- und Di-alkylaminocarbonyl, Mono- und Dialkylamino, Alkylsulfinyl oder Alkylsulfonyl substituiert ist, wobei bei den alkylhaltigen Resten solche mit 1 bis 4 C-Atomen bevorzugt sind.

Bevorzugte Verbindungen der Formel I sind solche, in denen

$R^3$, $R^a$, $R^b$ und A — wie oben definiert sind und n = null,

$R^1$ — H, $(C_1\text{-}C_4)$Alkyl, das unsubstituiert oder durch ein oder mehrere Halogenatome oder durch einen Rest aus der Gruppe $(C_1\text{-}C_3)$Alkoxy, $(C_1\text{-}C_3)$-Alkylthio, $(C_1\text{-}C_3)$-Alkylsulfonyl, $[(C_1\text{-}C_4)$Alkoxy]-carbonyl und CN substituiert ist, $(C_3\text{-}C_4)$Alkenyl, $(C_3\text{-}C_4)$-Alkinyl, $(C_1\text{-}C_4)$-Alkylsulfonyl, $(C_1\text{-}C_3)$Alkoxy oder $[(C_1\text{-}C_4)$Alkyl]-carbonyl und

$R^2$ — $(C_1\text{-}C_4)$Alkyl, das unsubstituiert oder durch 1 bis 3 Reste aus der Gruppe Halogen substituiert ist oder

$R^1$ und $R^2$ — gemeinsam eine Kette der Formel $-(CH_2)_o-$, wobei o 3 oder 4 bedeutet,

bedeuten.

Besonders bevorzugte Verbindungen der Formel (I) sind solche, worin

$R^1$ Wasserstoff, $(C_1-C_4)$Alkyl, Halo-$(C_1-C_4)$alkyl,
$R^2$ $(C_1-C_3)$Alkyl, Halo-$(C_1-C_4)$alkyl,
$R^3$ H, $(C_1-C_3)$Alkyl, $(C_1-C_3)$Alkoxy, Halogen oder $(C_1-C_3)$Alkylthio,
Z CH oder N,
X $(C_1-C_3)$Alkyl, $(C_1-C_3)$Alkoxy, $(C_1-C_3)$-Haloalkyl, $(C_1-C_3)$Haloalkoxy, $(C_1-C_3)$-Alkoxy-$(C_1-C_3)$alkyl oder $(C_1-C_3)$Alkoxy-$(C_1-C_3)$alkoxy und
Y Halogen, $(C_1-C_3)$Alkyl, $(C_1-C_3)$Alkoxy oder $(C_1-C_3)$Alkylthio, wobei die 3 letztgenannten Reste ein- oder mehrfach durch Halogen oder ein- oder zweifach durch Reste aus der Gruppe $(C_1-C_3)$Alkoxy und $(C_1-C_3)$Alkylthio substituiert sind,
oder einen Rest der Formel $NR^5R^6$, worin $R^5$ und $R^6$ die genannte Bedeutung haben, oder $(C_3-C_6)$Cycloalkyl, $(C_2-C_4)$Alkinyl, $(C_3-C_4)$Alkenyloxy oder $(C_3-C_4)$-Alkinyloxy

bedeuten.

X ist vorzugsweise $(C_1-C_2)$Alkyl, $(C_1-C_2)$Alkoxy, $OCF_2H$, $CF_3$ oder $OCH_2CF_3$ und
Y ist vorzugsweise $(C_1-C_2)$Alkyl, $(C_1-C_2)$Alkoxy, Halogen oder $OCF_2H$.
M ist vorzugsweise Na, Li, K, Ca oder eine Gruppe der Formel M1,

$NR^7R^8R^9R^{10}$ (M1)

worin $R^7$, $R^8$, $R^9$ und $R^{10}$ unabhängig voneinander H, $(C_1-C_{12})$Alkyl, $(C_3-C_4)$Alkenyl, $(C_3-C_4)$-Alkinyl, $(C_5-C_6)$Cycloalkyl, $[(C_1-C_4)$Alkoxy]-carbonyl-$(C_1-C_4)$alkyl, Hydroxy-$(C_1-C_4)$alkyl, Phenyl, Benzyl, 1-Phenylethyl oder 2-Phenylethyl bedeuten, oder

M ist vorzugsweise Piperidin, Pyrrolidin, Morpholin oder Pyridin.

Weiterer Gegenstand der vorliegenden Erfindung sind Verfahren zur Herstellung der Verbindungen der allgemeinen Formel (I), dadurch gekennzeichnet, daß man eine Verbindung der Formel (II),

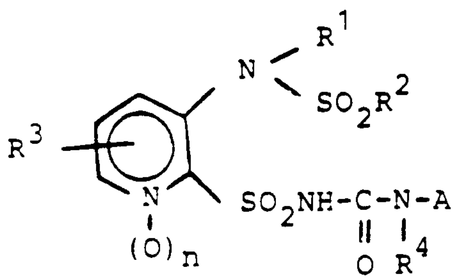

(II)

worin A, $R^1$, $R^2$, $R^3$, $R^4$ und n wie in Formel (I) nach Anspruch 1 definiert sind, mit einer Base der Formel (IIIa) oder (IIIb)

$M^{m+}(R^{11}O^-)_m$ (IIIa)

$(M^{m+})_pCO_3^{2-}$ (IIIb),

worin M und m wie in Formel (I) definiert sind und $R^{11}$ H oder einen aliphatischen oder aromatischen organischen Rest, vorzugsweise $(C_1-C_4)$Alkyl, und p für den Fall m = 1 die Zahl 2 und für den Fall m = 2 die Zahl 1 bedeuten,
umsetzt oder für den Fall, daß $R^{10}$ = H ist, mit einem Amin der Formel (IV),

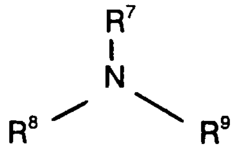

(IV)

worin $R^7$ bis $R^9$ wie bei Formel M1 definiert sind, umsetzt.

Man führt die Reaktion beispielsweise in Gegenwart von unter den Reaktionsbedingungen inerten

Lösungsmitteln durch. Geeignete Lösungsmittel sind anorganische Lösungsmittel wie Wasser oder organische Lösungsmittel, z.B. Alkohole, wie z.B. Methanol oder Ethanol, halogenierte Kohlenwasserstoffe, wie Dichlormethan, Ether, wie Tetrahydrofuran oder Dioxan, Ketone, wie Aceton oder MIBK (Methylisobutylketon), Amide wie DMF, Nitrile wie Acetonitril und Sulfoxide, wie DMSO. Die Reaktionstemperaturen sind meist zwischen 0 ° C und dem Siedepunkt des Lösungsmittels.
Ein analoges Verfahren mit Reaktionsbedingungen ist in WO 90/06308 beschrieben.

Die Produkte der Formel (I) können in praktisch quantitativer Ausbeute und guter Reinheit in manchen Fällen direkt durch Abfiltrieren oder, wenn sie gut löslich sind, nach destillativer Entfernung des Lösungsmittels isoliert werden.

Die Edukte der Formel (II) sind bekannt oder können analog bekannten Verfahren hergestellt werden. Ihre Synthese ist beispielsweise beschrieben in DE-A-4 000 503.
Die Basen der Formeln (IIIa) und (IIIb) sind in der Mehrzahl Standardreagenzien und zum Teil sogar in technischem Maßstab im Handel verfügbar. Es handelt sich beispielsweise um Alkali- und Erdalkalihydroxide, -alkoholate oder -carbonate, aber auch um quaternäre Ammoniumhydroxide, wie z.B. Tetrabutylammoniumhydroxid.

Die Basen der Formel (IV) umfassen Ammoniak, primäre, sekundäre und tertiäre Amine, wie z.B. Methylamin, Diethylamin, Triethylamin, Diethanolamin oder Benzylamin, aber auch heterocyclische Amine wie z.B. Pyridin, Pyrrolidin, Piperidin oder Morpholin.

Die Basen der Formeln (IIIa), (IIIb) oder (IV) werden beispielsweise in einer Menge von 0,5 bis 1,5 Mol, bevorzugt 0,9 bis 1,1 Mol, pro Mol Sulfonylharnstoff der Formel II eingesetzt.

Die erfindungsgemäßen Verbindungen der Formel (I) weisen eine ausgezeichnete herbizide Wirksamkeit gegen ein breites Spektrum wirtschaftlicher wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei können die Substanzen im Vorsaat-, Vorauflauf- oder Nachauflaufverfahren ausgebracht werden. 1m einzelnen seien beispielsweise einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung, z.B. durch Auslösen von Desikkation und Wuchstauchung, eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluent and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inerstoff noch Tenside ionischer und nichtionischer Art (Netzmittel, Dispergiermittel) z.B. polyoxyethylierte Alkylphenole, polyoxethylierte Fettalkohole und Fettamine, Fettalkoholpolyglykolethersulfate, Alkansulfonate oder Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden. Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 85 Gew.-%, meistens 5 bis 80 Gew.-% betragen. Staubförmige Formulierungen enthalten etwa 1 bis 25 Gew.-%, meistens 5 bis 20 Gew.-% an Wirkstoff, versprühbare Lösungen etwa 0,2 bis 20 Gew.-%, meistens 2 bis 20 Gew.-% Wirkstoff. Bei Granulaten hängt der Wirkstoffgehalt zum Teil davon ab, ob die wirksame Verbindung flüssig oder fest vorliegt. In der Regel liegt der Gehalt bei den in

Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Farb- und Trägerstoffe, Entschäumer, Verdunstungshemmer und den pH-Wert und die Viskosität beeinflussende Mittel.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate zur Boden- bzw. Streuapplikation sowie versprühbare Lösungen werden vor der Anwendung üblicherweise nicht m ehr mit weiteren inerten Stoffen verdünnt.

Die Applikation erfolgt beispielsweise auf die Pflanzen, Pflanzenteile, Pflanzensamen (Saatbeizung) oder die Anbaufläche.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

Auch Mischungen oder Mischformulierungen mit anderen Wirkstoffen, wie z.B. Insektiziden, Akariziden, Herbiziden, Safenern, Düngemitteln, Wachstumsregulatoren oder Fungiziden sind gegebenenfalls möglich.

A. Chemische Beispiele

a) 3-(4,6-Dimethoxypyrimidin-2-yl)-1-[3-(N-methyl-N-methylsulfonyl-amino)-pyrid-2-ylsulfonyl]-harnstoff-natriumsalz (zu Tabellenbeispiel 1)

3,0 g (0,13 Val) Natrium werden in 300 ml Methanol gelöst. Zu dieser Lösung gibt man bei Raumtemperatur 58,0 g (0,13 mol) 3-(4,6-Dimethoxypyrimidin-2-yl)-1-[3(N-methyl-N-methylsulfonyl-amino)-pyrid-2-ylsulfonyl]-harnstoff und erhitzt anschließend 30 min auf Rückfluß. Man kühlt ab, entfernt das Lösungsmittel im Vakuum und erhält so 60,7 g (100 %

d.Th.) des obengenannten Natriumsalzes vom Schmelzpunkt 203-205 ° C (Zers.).

b) 3-(4,6-Dimethoxypyrimidin-2-yl)-1-[3-(N-methyl-N-methylsulfonyl-amino)-pyrid-2-ylsulfonyl]-harnstoff-triethylammoniumsalz (zu Tabellenbeispiel 12)

Zu einer Lösung von 2,23 g (5 mmol) 3-(4,6-Dimethoxypyrimidin-2-yl)-1-[3-(N-methyl-N-methylsulfonylamino)-pyrid-2-ylsulfonyl]-harnstoff in 50 ml Dichlormethan gibt man 0,51 g (5 mmol) Triethylamin und erwärmt 30 min auf Rückfluß. Man kühlt ab, entfernt das Lösungsmittel im Vakuum und verreibt den Rückstand mit Diethylether. Man erhält 2,5 g (91 % d.Th.) des obengenannten Triethylammoniumsalzes vom Schmelzpunkt 99-101 ° C.

c) 3-(4,6-Dimethoxypyrimidin-2-yl)1-[3-(N-methyl-N-methylsulfonyl-amino)-pyrid-2-ylsulfonyl]-harnstoff-lithiumsalz (zu Tabellenbeispiel 2)

Zu einer Lösung von 0,21 g (5 mmol) Lithiumhydroxid (Monohydrat) in 50 ml Methanol gibt man 2,23 g (5 mmol) 3-(4,6-Dimethoxypyrimidin-2-yl)-1-[3-(N-methyl-N-methylsulfonyl-amino)-pyrid-2-ylsulfonyl]-harnstoff und erwärmt 30 min auf Rückfluß. Man kühlt ab, entfernt das Lösungsmittel im Vakuum und verreibt den Rückstand mit Diethylether. Man erhält 2,2 g (98 % d.Th.) des obengenannten Lithiumsalzes vom Schmelzpunkt 247-249 ° C (Zers.).

Die Verbindungen der nachfolgenden Tabelle 1 werden analog zu den Verfahren der Beispiele 1-3 erhalten.

Tabelle 1

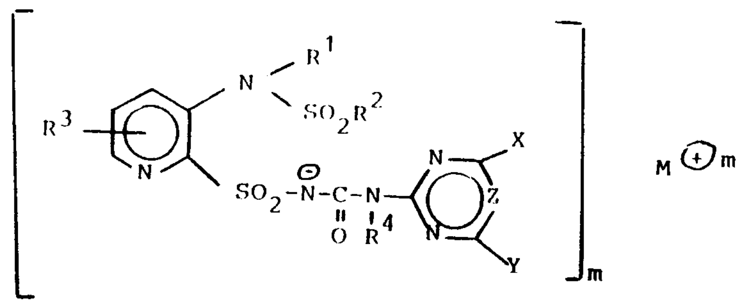

| Ver.Nr. | $R^1$ | $R^2$ | $R^3$ | $R^4$ | X | Y | Z | M | m | Smp. [°C] |
|---|---|---|---|---|---|---|---|---|---|---|
| 1 | $CH_3$ | $CH_3$ | H | H | $OCH_3$ | $OCH_3$ | CH | Na | 1 | 203-205 (Zers.) |
| 2 | " | " | " | " | " | " | " | Li | 1 | 247-249 " |
| 3 | " | " | " | " | " | " | " | K | 1 | 213-216 " |
| 4 | " | " | " | " | " | " | " | Mg | 2 | |
| 5 | " | " | " | " | " | " | " | Ca | 2 | |
| 6 | " | " | " | " | " | " | " | $NH_4$ | 1 | |
| 7 | " | " | " | " | " | " | " | $CH_3NH_3$ | 1 | |

| Ver.Nr. | $R^1$ | $R^2$ | $R^3$ | $R^4$ | X | Y | Z | M | m | Smp. [°C] |
|---|---|---|---|---|---|---|---|---|---|---|
| 8 | $CH_3$ | $CH_3$ | H | H | $OCH_3$ | $OCH_3$ | CH | $(CH_3)_2NH_2$ | 1 | |
| 9 | " | " | " | " | " | " | " | $(CH_3)_3NH$ | 1 | |
| 10 | " | " | " | " | " | " | " | $C_2H_5NH_3$ | 1 | |
| 11 | " | " | " | " | " | " | " | $(C_2H_5)_2NH_2$ | 1 | |
| 12 | " | " | " | " | " | " | " | $(C_2H_5)_3NH$ | 1 | 99-101 |
| 13 | " | " | " | " | " | " | " | $C_4H_9NH_3$ | 1 | 127-129 |
| 13a | " | " | " | " | " | " | " | $(C_4H_9)_4N$ | 1 | |
| 14 | " | " | " | " | " | " | " | $HOCH_2CH_2NH_3$ | 1 | |
| 15 | " | " | " | " | " | " | " | $(HOCH_2CH_2)_2NH_2$ | 1 | 65-68 |
| 16 | " | " | " | " | " | " | " | $(HOCH_2CH_2)_3NH$ | 1 | 142-144 |
| 17 | " | " | " | " | " | " | " | $C_{12}H_{25}NH_3$ | 1 | |
| 18 | " | " | " | " | " | " | " | $C_6H_5$-$CH_2NH_3$ | 1 | |
| 19 | " | " | " | " | " | " | " | $C_6H_5$-$CH_2CH_2NH_3$ | 1 | |

| Ver.Nr. | $R^1$ | $R^2$ | $R^3$ | $R^4$ | X | Y | Z | M | m | Smp. [°C] |
|---|---|---|---|---|---|---|---|---|---|---|
| 20 | $CH_3$ | $CH_3$ | H | H | $OCH_3$ | $OCH_3$ | CH | $C_6H_5$-$NH_3$ | 1 | |
| 21 | " | " | " | " | " | " | " | $H_5C_2OCO$-$CH_2NH_3$ | 1 | |
| 22 | " | " | " | " | " | " | " | 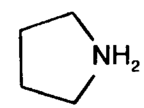 | 1 | |
| 23 | " | " | " | " | " | " | " | 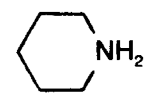 | 1 | |
| 24 | " | " | " | " | " | " | " | 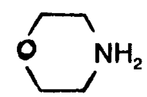 | 1 | |
| 25 | " | " | " | " | " | " | " | 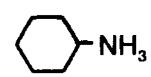 | 1 | |

| Ver.Nr. | $R^1$ | $R^2$ | $R^3$ | $R^4$ | X | Y | Z | M | m | Smp. [°C] |
|---|---|---|---|---|---|---|---|---|---|---|
| 26 | $CH_3$ | $CH_3$ | H | H | $OCH_3$ | $OCH_3$ | CH | 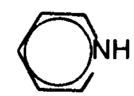 | 1 | |
| 27 | " | " | " | " | " | " | " | $H_2C=CH-CH_2NH_3$ | 1 | |
| 28 | " | " | " | " | " | " | " | $HC\equiv C-CH_2NH_3$ | 1 | |
| 29 | $CH_3$ | $CH_3$ | H | H | $OCH_3$ | $CH_3$ | CH | Na | 1 | 261-263 (Zers.) |
| 30 | " | " | " | " | " | " | " | K | 1 | |
| 31 | " | " | " | " | " | " | " | Mg | 2 | |
| 32 | " | " | " | " | " | " | " | Ca | 2 | |
| 33 | " | " | " | " | " | " | " | $NH_4$ | 1 | |
| 34 | " | " | " | " | " | " | " | $(C_2H_5)_3NH$ | 1 | |
| 35 | $CH_3$ | $CH_3$ | H | $CH_3$ | $OCH_3$ | $OCH_3$ | CH | Na | 1 | 199-201 (Zers.) |
| 36 | " | " | " | " | " | " | " | K | 1 | |
| 37 | " | " | " | " | " | " | " | Mg | 2 | |

| Ver.Nr. | $R^1$ | $R^2$ | $R^3$ | $R^4$ | X | Y | Z | M | m | Smp. [°C] |
|---|---|---|---|---|---|---|---|---|---|---|
| 38 | $CH_3$ | $CH_3$ | H | $CH_3$ | $OCH_3$ | $OCH_3$ | CH | Ca | 2 | |
| 39 | " | " | " | " | " | " | " | $NH_4$ | 1 | |
| 40 | " | " | " | " | " | " | " | $(C_2H_5)_3NH$ | 1 | |
| 41 | $CH_3$ | $CH_3$ | H | H | $OCH_3$ | $CH_3$ | N | Na | 1 | |
| 42 | " | " | " | " | " | " | " | K | 1 | |
| 43 | " | " | " | " | " | " | " | Mg | 2 | |
| 44 | " | " | " | " | " | " | " | Ca | 2 | |
| 45 | " | " | " | " | " | " | " | $NH_4$ | 1 | |
| 46 | " | " | " | " | " | " | " | $(C_2H_5)_3NH$ | 1 | |
| 47 | $CH_3$ | $CH_3$ | 6-$CH_3$ | H | $OCH_3$ | $OCH_3$ | CH | Na | 1 | 228-230 (Zers.) |
| 48 | " | " | " | " | " | " | " | K | 1 | |
| 49 | " | " | " | " | " | " | " | Mg | 2 | |
| 50 | " | " | " | " | " | " | " | Ca | 2 | |

| Ver.Nr. | $R^1$ | $R^2$ | $R^3$ | $R^4$ | X | Y | Z | M | m | Smp. [°C] |
|---|---|---|---|---|---|---|---|---|---|---|
| 51 | $CH_3$ | $CH_3$ | 6-$CH_3$ | H | $OCH_3$ | $OCH_3$ | CH | $NH_4$ | 1 | |
| 52 | " | " | " | " | " | " | " | $(C_2H_5)_3NH$ | 1 | Öl |
| 53 | $CH_3$ | $CH_3$ | 6-Cl | H | $OCH_3$ | $OCH_3$ | CH | Na | 1 | |
| 54 | " | " | 6-$OCH_3$ | " | " | " | " | Na | 1 | |
| 55 | " | " | 5-$CH_3$ | " | " | " | " | Na | 1 | |
| 56 | " | " | 4-$CH_3$ | " | " | " | " | Na | 1 | |
| 57 | $CH_3$ | $CH_3$ | H | H | Cl | $OCH_3$ | CH | Na | 1 | |
| 58 | " | " | " | " | $OCH_3$ | $OC_2H_5$ | CH | Na | 1 | |
| 59 | " | " | " | " | $OCH_3$ | $SCH_3$ | CH | Na | 1 | |
| 60 | " | " | " | " | F | $OCH_3$ | CH | Na | 1 | |
| 61 | " | " | " | " | $OCH_3$ | $OCH_3$ | N | Na | 1 | |
| 62 | " | " | " | " | $OCH_3$ | $CF_3$ | CH | Na | 1 | |
| 63 | " | " | " | $CH_3$ | $OCH_3$ | $CH_3$ | N | Na | 1 | |

| Ver.Nr. | $R^1$ | $R^2$ | $R^3$ | $R^4$ | X | Y | Z | M | m | Smp. [°C] |
|---|---|---|---|---|---|---|---|---|---|---|
| 64 | $CH_3$ | $CF_3$ | H | H | $OCH_3$ | $OCH_3$ | CH | Na | 1 | |
| 65 | " | $C_2H_5$ | " | " | " | " | " | Na | 1 | |
| 66 | $CHF_2$ | $CH_3$ | " | " | " | " | " | " | 1 | |
| 67 | $-C_2H_5$ | " | " | " | " | " | " | " | 1 | |
| 68 | $-CH(CH_3)_2$ | " | " | " | " | " | " | " | 1 | |
| 69 | $-C_3H_7$ | " | " | " | " | " | " | " | 1 | |
| 70 | $-CH_2CH=CH_2$ | | " | " | " | " | " | " | 1 | |
| 71 | $-CH_2C\equiv CH$ | " | " | " | " | " | " | " | 1 | |
| 72 | $-CH_2CH_2Cl$ | " | " | " | " | " | " | " | 1 | |
| 73 | $CH_2CH_2OCH_3$ | " | " | " | " | " | " | " | 1 | |
| 74 | $CH_3$ | $CH_3$ | " | " | $OCHF_2$ | $OCHF_2$ | CH | " | 1 | |

B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile

einer Verbindung der Formel (I), 64 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gew.-Teile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 °C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.

e) Ein in Wasser dispergierbares Granulat wird erhalten, indem man

| | | |
|---|---|---|
| 75 | Gewichtsteile | einer Verbindung der Formel (I), |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man

| | | |
|---|---|---|
| 25 | Gewichtsteil(e) | einer Verbindung der Formel (I) |
| 5 | " | 2,2'-dinaphthylmethan-6,6'-disulfonsaures Natrium, |
| 2 | " | oleoylmethyltaurinsaures Natrium, |
| 1 | " | Polyvinylalkohol, |
| 17 | " | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

g) Ein Extruder-Granulat erhält man, indem man 20 Gew.-Teile Wirkstoff, 3 Gew.-Teile ligninsulfonsaures Natrium, 1 Gew.-Teil Carboxymethylcellulose und 76 Gew.-Teile Kaolin vermischt, vermahlt und mit Wasser anfeuchtet. Dieses Gemisch wird extrudiert und anschließend in Luftstrom getrocknet.

## C. Biologische Beispiele

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte zeigen, weisen die erfindungsgemäßen Verbindungen eine

sehr gute herbizide Vorauflaufwirksamkeit gegen ein breites Spektrum von Ungräsern und Unkräutern auf. Im Vergleich zu den jeweiligen Sulfonylharnstoffen, welche den Salzen strukturell entsprechen, weisen z. B. die erfindungsgemäßen Salze der Beispiele 1, 2, 3, 12, 13, 15, 29 und 52 der Tabelle 1 zum Teil wesentlich bessere herbizide Wirkung bei monokotylen Unkrautarten wie Avena, Alopecurus, Echinochloa, Digitaria, Setaria, Cyperus, Bromus oder Sorhgum oder bei dikotylen Unkrautarten wie Galium, Viola, Veronica, Lamium, Stellaria, Amaranthus, Sinapis, Pharbitis oder Convolvulus auf.

2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine sehr gute herbizide Wirksamkeit gegen ein breites Spektrum wirtschaftlich wichtiger Ungräser und Unkräuter auf. Im Vergleich zu den jeweiligen Sulfonylharnstoffen, welche den Salzen strukturell entsprechen, weisen z. B. die erfindungsgemäßen Salze der Beispiele 1, 2, 3, 12, 13, 15, 29 und 52 der Tabelle 1 zum Teil wesentlich bessere herbizide Wirkung bei monokotylen Unkrautarten wie Avena, Alopecurus, Echinochloa, Digitaria, Setaria, Cyperus, Bromus oder Sorhgum oder bei dikotylen Unkrautarten wie Galium, Viola, Veronica, Lamium, Stellaria, Amaranthus, Sinapis, Pharbitis oder Convolvulus auf.

3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel (I) weisen somit eine hohe Selektivität bei Anwendung zur Bekämpfung von unerwünschtem Pflanzenwuchs in landwirtschaftlichen Kulturen auf. Die Verbindungen der Beispiele 1, 2, 3, 12, 13, 15, 29 und 52 der Tabelle 1 zeigten beispielsweise gute Selektivitäten in Getreidekulturen wie Weizen und Gerste sowie in Reis, Soja und Mais.

**Patentansprüche**

**1.** Verbindungen der Formel (I)

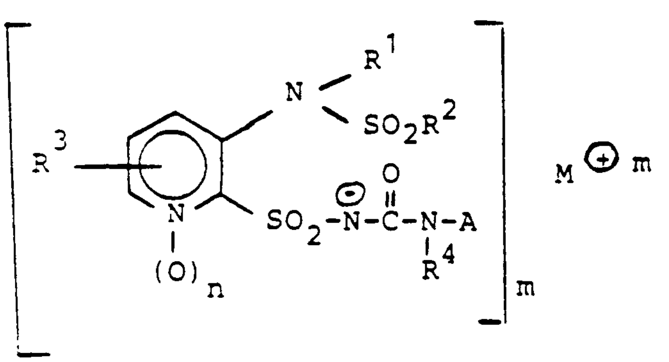

(I)

worin

- $R^1$ H, $(C_1\text{-}C_6)$Alkyl, das unsubstituiert oder durch ein oder mehrere Reste aus der Gruppe Halogen, $(C_1\text{-}C_4)$Alkoxy, $(C_1\text{-}C_4)$Alkylthio, $(C_1\text{-}C_4)$Alkylsulfinyl, $(C_1\text{-}C_4)$-Alkylsulfonyl, $[(C_1\text{-}C_4)$Alkoxy]-carbonyl und CN substituiert ist, $(C_3\text{-}C_6)$Alkenyl, das unsubstituiert oder durch ein oder mehrere Halogenatome substituiert ist, $(C_3\text{-}C_6)$-Alkinyl, das unsubstituiert oder durch ein oder mehrere Halogenatome substituiert ist, $(C_1\text{-}C_4)$-Alkylsulfonyl, das unsubstituiert oder durch ein oder mehrere Halogenatome substituiert ist, Phenylsulfonyl, wobei der Phenylrest unsubstituiert oder durch ein oder mehrere Reste aus der Gruppe Halogen, $(C_1\text{-}C_4)$Alkyl und $(C_1\text{-}C_4)$Alkoxy substituiert ist, $(C_1\text{-}C_4)$Alkoxy oder $[(C_1\text{-}C_4)$Alkyl]-carbonyl, das unsubstituiert oder durch ein oder mehrere Halogenatome substituiert ist,
- $R^2$ $(C_1\text{-}C_4)$Alkyl, das unsubstituiert oder durch ein oder mehrere Halogenatome substituiert ist, Phenyl, wobei der Phenylrest unsubstituiert oder durch ein oder mehrere Reste aus der Gruppe Halogen, $(C_1\text{-}C_4)$Alkyl und $(C_1\text{-}C_4)$Alkoxy substituiert ist, oder Di-$[(C_1\text{-}C_4)$alkyl]amino oder
- $R^1$ und $R^2$ gemeinsam eine Kette der Formel $-(CH_2)_o-$, wobei die Kette noch durch bis 4 $(C_1\text{-}C_3)$-Alkylreste substituiert sein kann und o 3 oder 4 bedeutet,
- $R^3$ H, $(C_1\text{-}C_4)$Alkyl, $(C_1\text{-}C_3)$Haloalkyl, Halogen, $NO_2$, CN, $(C_1\text{-}C_3)$Alkoxy, $(C_1\text{-}C_3)$-Haloalkoxy, $(C_1\text{-}C_3)$Alkylthio, $(C_1\text{-}C_3)$Alkoxy-$(C_1\text{-}C_3)$alkyl, $[(C_1\text{-}C_3)$Alkoxy]-carbonyl, $(C_1\text{-}C_3)$Alkylamino, Di$[(C_1\text{-}C_3)$alkyl]-amino, $(C_1\text{-}C_3)$-Alkylsulfinyl, $(C_1\text{-}C_3)$Alkylsulfonyl, $SO_2NR^aR^b$ oder $C(O)NR^aR^b$,
- $R^a,R^b$ unabhängig voneinander H, $(C_1\text{-}C_3)$Alkyl, $(C_3\text{-}C_4)$Alkenyl oder Propargyl oder zusammen $-(CH_2)_4-$, $-(CH_2)_5-$ oder $-CH_2CH_2OCH_2CH_2-$
- $R^4$ H oder $CH_3$,
- n null oder 1,
- m 1 oder 2,
- A einen Rest der Formel

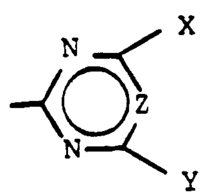

- X, Y unabhängig voneinander H, Halogen, $(C_1\text{-}C_3)$Alkyl, $(C_1\text{-}C_3)$Alkoxy oder $(C_1\text{-}C_3)$-Alkylthio, wobei die vorgenannten alkylhaltigen Reste unsubstituiert oder ein- oder mehrfach durch Halogen oder ein- oder zweifach durch $(C_1\text{-}C_3)$Alkoxy oder $(C_1\text{-}C_3)$-Alkylthio substituiert sind, ferner einen Rest der Formel $NR^5R^6$, $(C_3\text{-}C_6)$-Cycloalkyl, $(C_2\text{-}C_4)$Alkenyl, $(C_2\text{-}C_4)$Alkinyl, $(C_3\text{-}C_4)$-Alkenyloxy oder $(C_3\text{-}C_4)$Alkinyloxy,
- Z CH oder N,
- $R^5$ und $R^6$ unabhängig voneinander H, $(C_1\text{-}C_3)$Alkyl oder $(C_3\text{-}C_4)$Alkenyl und
- M ein Atom aus der Gruppe der Alkalimetalle, Erdalkalimetalle oder eine Gruppe der Formel M1,

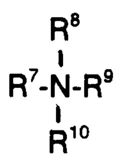

(M1)

worin $R^7$, $R^8$, $R^9$ und $R^{10}$ unabhängig voneinander für H, $(C_1\text{-}C_{12})$Alkyl, $(C_3\text{-}C_6)$-Alkenyl, $(C_3\text{-}C_6)$Alkinyl, $(C_3\text{-}C_8)$-Cycloalkyl oder Phenyl, wobei die 5 letztgenannten Reste jeweils unabhängig voneinander unsubstituiert oder durch ein oder mehrere Reste aus der Gruppe Halogen, $(C_1\text{-}C_4)$Alkyl, Hydroxy, $(C_1\text{-}C_4)$Alkoxy, Thiol, $(C_1\text{-}C_4)$-Alkylthio, $[(C_1\text{-}C_4)$Alkoxy]-carbonyl und gegebenenfalls substituiertes Phenyl substituiert sind, stehen oder zwei der Reste $R^7$ bis $R^{10}$ gemeinsam für eine gesättigte oder ungesättigte Kette von 3-7 C-Atomen, wobei 1-2 C-Atome durch Atome aus der Gruppe O, N oder S ersetzt sein können und die Kette durch 1-3 $(C_1-C_4)$Alkylreste substituiert sein kann, stehen und die übrigen zwei Reste die vorher genannten Bedeutungen für einzelne Reste $R^7$ bis $R^{10}$ haben,

bedeuten.

**2.** Verbindungen nach Anspruch 1, dadurch gekennzeichnet, daß

$R^3$, $R^a$, $R^b$ und A wie in Anspruch 1 definiert sind und n = null,

$R^1$ H, $(C_1-C_4)$Alkyl, das unsubstituiert oder durch ein oder mehrere Halogenatome oder durch einen Rest aus der Gruppe $(C_1-C_3)$Alkoxy, $(C_1-C_3)$-Alkylthio, $(C_1-C_3)$-Alkylsulfonyl, $[(C_1-C_4)$Alkoxy]-carbonyl und CN substituiert ist, $(C_3-C_4)$-Alkenyl, $(C_3-C_4)$Alkinyl, $(C_1-C_4)$-Alkylsulfonyl, $(C_1-C_3)$Alkoxy oder $[(C_1-C_4)$Alkyl]-carbonyl und

$R^2$ $(C_1-C_4)$Alkyl, das unsubstituiert oder durch 1 bis 3 Reste aus der Gruppe Halogen substituiert ist oder

$R^1$ und $R^2$ gemeinsam eine Kette der Formel $-(CH_2)_o-$, wobei o 3 oder 4 bedeutet, bedeuten.

**3.** Verbindung nach Anspruch 1 oder 2, dadurch gekennzeichnet, daß

$R^1$ Wasserstoff, $(C_1-C_4)$Alkyl, Halo-$(C_1-C_4)$alkyl,

$R^2$ $(C_1-C_3)$Alkyl, Halo-$(C_1-C_4)$alkyl,

$R^3$ H, $(C_1-C_3)$Alkyl, $(C_1-C_3)$Alkoxy, Halogen oder $(C_1-C_3)$Alkylthio,

Z CH oder N,

X $(C_1-C_3)$Alkyl, $(C_1-C_3)$Alkoxy, $(C_1-C_3)$-Haloalkyl, $(C_1-C_3)$Haloalkoxy, $(C_1-C_3)$Alkoxy-$(C_1-C_3)$alkyl oder $(C_1-C_3)$Alkoxy-$(C_1-C_3)$alkoxy und

Y Halogen, $(C_1-C_3)$Alkyl, $(C_1-C_3)$Alkoxy oder $(C_1-C_3)$Alkylthio, wobei die 3 letztgenannten Reste ein- oder mehrfach durch Halogen oder ein- oder zweifach durch Reste aus der Gruppe $(C_1-C_3)$Alkoxy und $(C_1-C_3)$Alkylthio substituiert sind, oder einen Rest der Formel $NR^5R^6$, worin $R^5$ und $R^6$ die genannte Bedeutung haben, oder $(C_3-C_6)$Cycloalkyl, $(C_2-C_4)$Alkinyl, $(C_3-C_4)$-Alkenyloxy oder $(C_3-C_4)$-Alkinyloxy bedeuten.

**4.** Verbindung nach einem der Ansprüche 1 bis 3, dadurch gekennzeichnet, daß X $(C_1-C_2)$Alkyl, $(C_1-C_2)$-Alkoxy, $OCF_2H$, $CF_3$ oder $OCH_2CF_3$ und Y $(C_1-C_2)$Alkyl, $(C_1-C_2)$Alkoxy, Halogen oder $OCF_2H$ bedeuten.

**5.** Verbindung nach einem der Ansprüche 1 bis 4, dadurch gekennzeichnet, daß

M Na, Li, K, Ca oder eine Gruppe der Formel M1,

$NR^7R^8R^9R^{10}$ (M1)

worin $R^7$, $R^8$, $R^9$ und $R^{10}$ unabhängig voneinander H, $(C_1-C_{12})$Alkyl, $(C_3-C_4)$Alkenyl, $(C_3-C_4)$-Alkinyl, $(C_5-C_6)$Cycloalkyl, $[(C_1-C_4)$Alkoxy]-carbonyl-$(C_1-C_4)$alkyl, Hydroxy-$(C_1-C_4)$alkyl, Phenyl, Benzyl, 1-Phenylethyl oder 2-Phenylethyl bedeuten, oder

M Piperidin, Pyrrolidin, Morpholin oder Pyridin

bedeutet.

**6.** Verfahren zur Herstellung einer Verbindung der Formel (I) gemäß Anspruch 1, dadurch gekennzeichnet, daß man eine Verbindung der Formel (II),

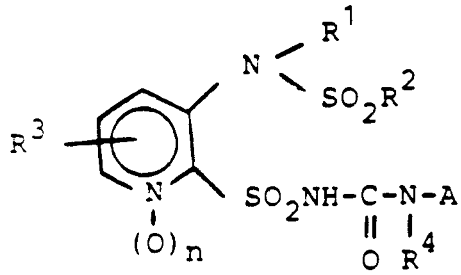

(II)

worin A, $R^1$, $R^2$, $R^3$, $R^4$ und n wie in Formel (I) nach Anspruch 1 definiert sind, mit einer Base der Formel (IIIa) oder (IIIb)

$M^{m+} (R^{11}O^-)_m$ (IIIa)

$(M^{m+})_p CO_3^{2-}$ (IIIb)

worin M und m wie in Formel (I) nach Anspruch 1 definiert sind und $R^{11}$ H oder einen aliphatischen oder aromatischen organischen Rest und p für den Fall m = 1 die Zahl 2 und für den Fall m = 2 die Zahlt 1 bedeuten, umsetzt oder für den Fall, daß $R^{10}$ = H ist, mit einem Amin der Formel (IV),

$NR^7R^8R^9$ (IV)

worin $R^7$ bis $R^9$ wie bei Formel (I) nach Anspruch 1 definiert sind, umsetzt.

**7.** Verwendung der Verbindungen der Formel (I) nach einem der Ansprüche 1 bis 5 als Herbizide oder Pflanzenwachstumsregulatoren.

**8.** Herbizide und pflanzenwachstumsregulierende Mittel, dadurch gekennzeichnet, daß sie eine Verbindung der Formel (I) nach einem der Ansprüche 1 bis 5 und übliche Formulierungshilfsmittel enthalten.

**9.** Verfahren zur selektiven oder unselektiven Bekämpfung von Schadpflanzen oder zur Wachstumsregulierung von Pflanzen, dadurch gekennzeichnet, daß man eine oder mehrere Verbindungen der Formel (I) nach einem der Ansprüche 1 bis 5 auf die Pflanzen, Pflanzenteile, Pflanzensamen oder die Anbaufläche appliziert.

**Patentansprüche für folgenden Vertragsstaat : ES**

**1.** Verwendung von Verbindungen der Formel (I),

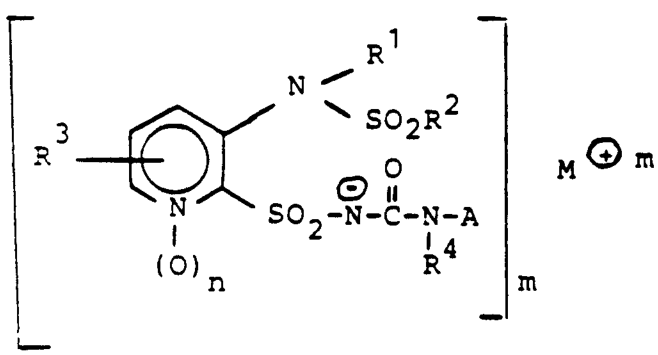

(I)

worin

$R^1$ H, $(C_1-C_6)$Alkyl, das unsubstituiert oder durch ein oder mehrere Reste aus der Gruppe Halogen, $(C_1-C_4)$Alkoxy, $(C_1-C_4)$Alkylthio, $(C_1-C_4)$Alkylsulfinyl, $(C_1-C_4)$-Alkylsulfonyl, $[(C_1-C_4)$Alkoxy]-carbonyl und CN substituiert ist, $(C_3-C_6)$Alkenyl, das unsub-

stituiert oder durch ein oder mehrere Halogenatome substituiert ist, ($C_3$-$C_6$)-Alkinyl, das unsubstituiert oder durch ein oder mehrere Halogenatome substituiert ist, ($C_1$-$C_4$)-Alkylsulfonyl, das unsubstituiert oder durch ein oder mehrere Halogenatome substituiert ist, Phenylsulfonyl, wobei der Phenylrest unsubstituiert oder durch ein oder mehrere Reste aus der Gruppe Halogen, ($C_1$-$C_4$)Alkyl und ($C_1$-$C_4$)Alkoxy substituiert ist, ($C_1$-$C_4$)Alkoxy oder [($C_1$-$C_4$)Alkyl]-carbonyl, da unsubstituiert oder durch ein oder mehrere Halogenatome substituiert ist,

$R^2$ ($C_1$-$C_4$)Alkyl, das unsubstituiert oder durch ein oder mehrere Halogenatome substituiert ist, Phenyl, wobei der Phenylrest unsubstituiert oder durch ein oder mehrere Reste aus der Gruppe Halogen, ($C_1$-$C_4$)Alkyl und ($C_1$-$C_4$)Alkoxy substituiert ist, oder Di-[($C_1$-$C_4$)alkyl]amino oder

$R^1$ und $R^2$ gemeinsam eine Kette der Formel $-(CN_2)_o-$, wobei die Kette noch durch bis 4 ($C_1$-$C_3$)-Alkylreste substituiert sein kann und o 3 oder 4 bedeutet,

$R^3$ H, ($C_1$-$C_4$)Alkyl, ($C_1$-$C_3$)Haloalkyl, Halogen, $NO_2$, CN, ($C_1$-$C_3$)Alkoxy, ($C_1$-$C_3$)-Haloalkoxy, ($C_1$-$C_3$)Alkylthio, ($C_1$-$C_3$)Alkoxy-($C_1$-$C_3$)alkyl, [($C_1$-$C_3$)Alkoxy]-carbonyl, ($C_1$-$C_3$)Alkylamino, Di[($C_1$-$C_3$)alkyl]-amino, ($C_1$-$C_3$)Alkylsulfinyl, ($C_1$-$C_3$)Alkylsulfonyl, $SO_2NR^aR^b$ oder $C(O)NR^aR^b$,

$R^a$,$R^b$ unabhängig voneinander H, ($C_1$-$C_3$)Alkyl, ($C_3$-$C_4$)Alkenyl oder Propargyl oder zusammen $-(CH_2)_4-$, $-(CH_2)_5-$ oder $-CH_2CH_2OCH_2CH_2-$

$R^4$ H oder $CH_3$,

n null oder 1,

m 1 oder 2,

A einen Rest der Formel

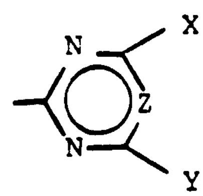

X, Y unabhängig voneinander H, Halogen, ($C_1$-$C_3$)Alkyl, ($C_1$-$C_3$)Alkoxy oder ($C_1$-$C_3$)-Alkylthio, wobei die vorgenannten alkylhaltigen Reste unsubstituiert oder ein- oder mehrfach durch Halogen oder ein- oder zweifach durch ($C_1$-$C_3$)Alkoxy oder ($C_1$-$C_3$)-Alkylthio substituiert sind, ferner einen Rest der Formel $NR^5R^6$, ($C_3$-$C_6$)-Cycloalkyl, ($C_2$-$C_4$)Alkenyl, ($C_2$-$C_4$)Alkinyl, ($C_3$-$C_4$)-Alkenyloxy oder ($C_3$-$C_4$)Alkinyloxy,

Z CH oder N,

$R^5$ und $R^6$ unabhängig voneinander H, ($C_1$-$C_3$)Alkyl oder ($C_3$-$C_4$)Alkenyl und

M ein Atom aus der Gruppe der Alkalimetalle, Erdalkalimetalle oder eine Gruppe der Formel M1,

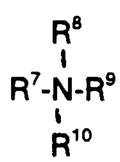

(M1)

worin $R^7$, $R^8$, $R^9$ und $R^{10}$ unabhängig voneinander für H, ($C_1$-$C_{12}$)Alkyl, ($C_3$-$C_6$)-Alkenyl, ($C_3$-$C_6$)Alkinyl, ($C_3$-$C_8$)-Cycloalkyl oder Phenyl, wobei die 5 letztgenannten Reste jeweils unabhängig voneinander unsubstituiert oder durch ein oder mehrere Reste aus der Gruppe Halogen, ($C_1$-$C_4$)Alkyl, Hydroxy, ($C_1$-$C_4$)Alkoxy, Thiol, ($C_1$-$C_4$)-Alkylthio, [($C_1$-$C_4$)Alkoxy]-carbonyl und gegebenenfalls substituiertes Phenyl substituiert sind, stehen oder zwei der Reste $R^7$ bis $R^{10}$ gemeinsam für eine gesättigte oder ungesättigte Kette von 3-7 C-Atomen, wobei 1-2 C-Atome durch Atome aus der Gruppe O, N oder S ersetzt sein können und die Kette durch 1-3 ($C_1$-$C_4$)Alkylreste substituiert sein kann, stehen und die übrigen zwei Reste die vorher genannten

Bedeutungen für einzelne Reste $R^7$ bis $R^{10}$ haben,
bedeuten, als Herbizide oder Pflanzenwachstumsregulatoren.

**2.** Verwendung nach Anspruch 1, dadurch gekennzeichnet, daß

| | |
|---|---|
| $R^3$, $R^a$, $R^b$ und A | wie in Anspruch 1 definiert sind und n = null, |
| $R^1$ | H, $(C_1-C_4)$Alkyl, das unsubstituiert oder durch ein oder mehrere Halogenatome oder durch einen Rest aus der Gruppe $(C_1-C_3)$Alkoxy, $(C_1-C_3)$-Alkylthio, $(C_1-C_3)$-Alkylsulfonyl, $[(C_1-C_4)$Alkoxy]-carbonyl und CN substituiert ist, $(C_3-C_4)$-Alkenyl, $(C_3-C_4)$Alkinyl, $(C_1-C_4)$-Alkylsulfonyl, $(C_1-C_3)$Alkoxy oder $[(C_1-C_4)$Alkyl]-carbonyl und |
| $R^2$ | $(C_1-C_4)$Alkyl, das unsubstituiert oder durch 1 bis 3 Reste aus der Gruppe Halogen substituiert ist oder |
| $R^1$ und $R^2$ | gemeinsam eine Kette der Formel $-(CH_2)_o-$, wobei o 3 oder 4 bedeutet, bedeuten. |

**3.** Verwendung nach Anspruch 1 oder 2, dadurch gekennzeichnet, daß

| | |
|---|---|
| $R^1$ | Wasserstoff, $(C_1-C_4)$Alkyl, Halo-$(C_1-C_4)$alkyl, |
| $R^2$ | $(C_1-C_3)$Alkyl, Halo-$(C_1-C_4)$alkyl, |
| $R^3$ | H, $(C_1-C_3)$Alkyl, $(C_1-C_3)$Alkoxy, Halogen oder $(C_1-C_3)$Alkylthio, |
| Z | CH oder N, |
| X | $(C_1-C_3)$Alkyl, $(C_1-C_3)$Alkoxy, $(C_1-C_3)$-Haloalkyl, $(C_1-C_3)$Haloalkoxy, $(C_1-C_3)$-Alkoxy-$(C_1-C_3)$-alkyl oder $(C_1-C_3)$Alkoxy-$(C_1-C_3)$alkoxy und |
| Y | Halogen, $(C_1-C_3)$Alkyl, $(C_1-C_3)$Alkoxy oder $(C_1-C_3)$Alkylthio, wobei die 3 letztgenannten Reste ein- oder mehrfach durch Halogen oder ein- oder zweifach durch Reste aus der Gruppe $(C_1-C_3)$Alkoxy und $(C_1-C_3)$Alkylthio substituiert sind, oder einen Rest der Formel $NR^5R^6$, worin $R^5$ und $R^6$ die genannte Bedeutung haben, oder $(C_3-C_6)$Cycloalkyl, $(C_2-C_4)$Alkinyl, $(C_3-C_4)$-Alkenyloxy oder $(C_3-C_4)$-Alkinyloxy bedeuten. |

**4.** Verwendung nach einem der Ansprüche 1 bis 3, dadurch gekennzeichnet, daß X $(C_1-C_2)$Alkyl, $(C_1-C_2)$-Alkoxy, $OCF_2H$, $CF_3$ oder $OCH_2CF_3$ und Y $(C_1-C_2)$Alkyl, $(C_1-C_2)$Alkoxy, Halogen oder $OCF_2H$ bedeuten.

**5.** Verwendung nach einem der Ansprüche 1 bis 4, dadurch gekennzeichnet, daß

M Na, Li, K, Ca oder eine Gruppe der Formel M1,

$NR^7R^8R^9R^{10}$ (M1)

worin $R^7$, $R^8$, $R^9$ und $R^{10}$ unabhängig voneinander H, $(C_1-C_{12})$Alkyl, $(C_3-C_4)$Alkenyl, $(C_3-C_4)$-Alkinyl, $(C_5-C_6)$Cycloalkyl, $[(C_1-C_4)$Alkoxy]-carbonyl-$(C_1-C_4)$alkyl, Hydroxy-$(C_1-C_4)$alkyl, Phenyl, Benzyl, 1-Phenylethyl oder 2-Phenylethyl bedeuten, oder

M Piperidin, Pyrrolidin, Morpholin oder Pyridin

bedeutet.

**6.** Verfahren zur Herstellung einer Verbindung der Formel (I) gemäß Anspruch 1, dadurch gekennzeichnet, daß man eine Verbindung der Formel (II),

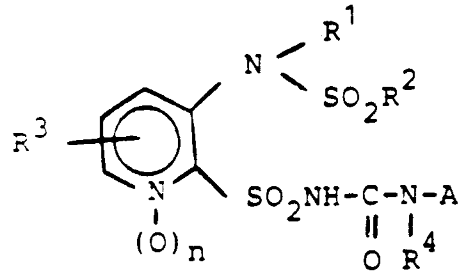

(II)

worin A, $R^1$, $R^2$, $R^3$, $R^4$ und n wie in Formel (I) nach Anspruch 1 definiert sind, mit einer Base der

Formel (IIIa) oder (IIIb)

$M^{m+}$ $(R^{11}O^{-})_m$ (IIIa)

$(M^{m+})_pCO_3^{2-}$ (IIIb)

worin M und m wie in Formel (I) nach Anspruch 1 definiert sind und $R^{11}$ H oder einen aliphatischen oder aromatischen organischen Rest und p für den Fall m = 1 die Zahl 2 und für den Fall m = 2 die Zahl 1 bedeuten, umsetzt oder für den Fall, daß $R^{10}$ = H ist, mit einem Amin der Formel (IV),

$NR^7R^8R^9$ (IV)

worin $R^7$ bis $R^9$ wie bei Formel (I) nach Anspruch 1 definiert sind, umsetzt.

**7.** Verfahren zur Herstellung eines herbiziden und pflanzenwachstumsregulierende Mittels, dadurch gekennzeichnet, daß man eine Verbindung der Formel (I) gemäß einem der Ansprüche 1 bis 5 zusammen mit üblichen Formulierungshilfsmitteln in eine für den Pflanzenschutz geeignete Anwendungsform bringt.

**8.** Verfahren zur selektiven oder unselektiven Bekämpfung von Schadpflanzen oder zur Wachstumsregulierung von Pflanzen, dadurch gekennzeichnet, daß man eine oder mehrere Verbindungen der Formel (I) nach einem der Ansprüche 1 bis 5 auf die Pflanzen, Pflanzenteile, Pflanzensamen oder die Anbaufläche appliziert.

Europäisches Patentamt

# EUROPÄISCHER RECHERCHENBERICHT

Nummer der Anmeldung

EP 92 11 1196

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,P,<br>D | DE-A-4 000 503 (HOECHST AKTIENGESELLSCHAFT)<br>*Tabelle 1, Verbindungen 411-494; Tabelle 2, Verbindungen 538-541*<br>* Seite 3, Zeile 18 - Zeile 22; Ansprüche 1-6 *<br>* Seite 4, Zeile 62 - Zeile 66 *<br>--- | 1-9 | C07D521/00<br>A01N47/36 |
| X,P | EP-A-0 451 468 (ISHIHARA SANGYO KAISHA, LTD)<br>*Tabelle 2, Verbindung 17; Formulierung Beispiel 1*<br>* Seite 2, Zeile 2 - Zeile 4; Ansprüche 1-11; Tabelle 3 *<br>--- | 1-9 | |
| Y,P,<br>D<br><br><br>D | WO-A-9 110 660 (HOECHST AKTIENGESELLSCHAFT)<br>* Seite 4 *<br>* Seite 8; Ansprüche 1,6,7 *<br>& DE-A-4 000 503<br>--- | 1,7-9 | |
| | | | RECHERCHIERTE SACHGEBIETE (Int. Cl.5) |
| Y,D | WO-A-9 006 308 (E. I. DU PONT DE NEMOURS AND COMPANY)<br>* das ganze Dokument *<br>----- | 1,7-9 | C07D |

Der vorliegende Recherchenbericht wurde für alle Patentansprüche erstellt

| Recherchenort | Abschlußdatum der Recherche | Prüfer |
|---|---|---|
| DEN HAAG | 02 OKTOBER 1992 | P. BOSMA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)